# EUROPEAN PATENT SPECIFICATION

(11) **EP 3 479 837 B1**
(45) Date of publication and mention of the grant of the patent: **06.09.2023**
(21) Application number: 17820253.7
(22) Date of filing: 29.06.2017
(51) Int. Cl.: A61K 35/747, A23C 9/12, A23L 33/135, A61P 7/06, A61P 13/12

(54) **RENAL ANEMIA AMELIORATING COMPOSITION**
ZUSAMMENSETZUNG ZUR LINDERUNG VON RENALER ANÄMIE
COMPOSITION AMÉLIORANT L'ANÉMIE RÉNALE

(30) Priority: 30.06.2016 JP 2016130811
(43) Date of publication of application: 08.05.2019
(73) Proprietor: Asahi Group Holdings, Ltd., Tokyo 130-8602 (JP); Tokushima University, Tokushima 770-8501 (JP)
(72) Inventor: ROKUTAN Kazuhito, Tokushima-shi Tokushima 770-8501 (JP); NISHIDA Kensei, Tokushima-shi Tokushima 770-8501 (JP); KUWANO Yuki, Tokushima-shi Tokushima 770-8501 (JP); FUJIWARA Shigeru, Sagamihara-shi Kanagawa 252-0206 (JP); SUGAWARA Tomonori, Sagamihara-shi Kanagawa 252-0206 (JP); AOKI Yumeko, Sagamihara-shi Kanagawa 252-0206 (JP); SAWADA Daisuke, Sagamihara-shi Kanagawa 252-0206 (JP)
(74) Representative: D Young & Co LLP
(86) International application number: PCT/JP2017/023851
(87) International publication number: WO 2018/003899

(56) References cited:
- WO-A1-2010/038714
- CN-A- 104 000 183
- JP-A- 2014 113 137
- JP-A- 2015 108 010
- US-A1- 2004 258 779
- US-A1- 2015 283 187
- SHARIATY Z. et al.: "The effects of probiotic supplement on hemoglobin in chronic renal failure patients under hemodialysis:A randomized clinical trial", Journal of Research in Medical Sciences, vol. 22, no. 3, June 2017 (2017-06), pages 1-7, XP055569399,

## Description

### [Technical Field]

The present invention concerns a composition for use in ameliorating renal anemia in a subject having decreased hematopoietic function of the kidney or a subject having decreased hemoglobin comprising, as an active ingredient, lactic acid bacteria according to the invention and/or a treated product thereof according to the invention.

### [Background Art]

Blood hemoglobin has been known as an indicator of anemia, and iron deficiency (absorption suppression) or renal cause (inhibition of secretion of hematopoietic hormones from the kidney) has been known as the causes of anemia. In the prior art, promotion of absorbed iron by administration of lactic acid bacteria and improvement of renal function related to excretion have been reported.

Specifically, Patent Literature 1 describes a composition for treating iron deficiency anemia comprising, as active ingredients, fermented milk which is obtained by fermentation using lactic acid bacteria belonging to *Lactobacillus acidophilus,* and iron salt.

Patent Literature 2 describes a composition in which probiotic bacteria such as *Lactobacillus* increase the excretory function in animal and human kidneys.

Patent Literature 3 describes that living cells of *Lactobacillus gasseri* suppress decrease or deterioration of renal function (metabolic function of the kidney).

Patent Literature 4 describes that certain strains of *Lactobacillus plantarum* increase the absorption of metal ions in a mammal.

Non-Patent Literature 1 describes that fermented milk obtained by using lactic acid bacteria belonging to *Lactobacillus bulgaricus* and *Streptococcus thermophilus* suppresses the absorption of iron, and thus is not effective for iron deficiency anemia.
WO2010/038714 discloses lactic acid bacteria having oxalic acid-degrading activity several times higher than those of conventionally known lactic acid bacteria having an oxalic acid-degrading activity, and uses thereof.
CN104000183 discloses medicinal and edible formula foodstuffs, and particularly relates to food for curing anemia.

### [Citation List]

### [Patent Literature]

Patent Literature 1: JP Patent Publication No. H07-053391 A
Patent Literature 2: JP Patent Publication No. 2007-507526 A
Patent Literature 3: JP Patent Publication No. 2014-55195 A
Patent Literature 4: JP Patent Publication No. 2008-544753 A

### [Non-Patent Literature]

Non-Patent Literature 1: G. Schaafsma et al., Neth. Milk Dairy J., Vol.42, 135-146, 1988

### [Summary of the Invention]

### [Objects to Be Attained by the Invention]

The present invention provides a composition for use in ameliorating renal anemia in a subject having decreased hematopoietic function of the kidney or a subject having decreased hemoglobin comprising, as an active ingredient, lactic acid bacteria, wherein the lactic acid bacteria are Lactobacillus gasseri strain CP2305 (Accession Number: FERM BP-11331), Lactobacillus gasseri strain CP2305s (Accession Number: NITE BP-1405), or Lactobacillus amylovorus strain CP1563 (Accession Number: FERM BP-11255), and/or a treated product thereof; wherein the treated product thereof comprises disrupted cells or cell-free extract of the lactic acid bacteria.

The composition for use according to the invention is effective for ameliorating renal anemia in subjects having decreased hematopoietic function of the kidney.

### [Means for Attaining the Objects]

The present inventors have conducted concentrated studies in order to attain the above objects. As a result, they found that renal anemia could be ameliorated in a subject by administering lactic acid bacteria belonging to *Lactobacillus* (*Lactobacillus gasseri* or *Lactobacillus amylovorus,* in particular) thereto. Specifically, they found that hemoglobin, erythrocytes and hematocrit could be increased or the decrease thereof could be prevented by the administration of the lactic acid bacteria.

Described herein is a composition for use in ameliorating renal anemia according to the invention. The composition according to the invention is particularly effective for ameliorating renal anemia in a subject having decreased hematopoietic function of the kidney. In addition, lactic acid bacteria as active ingredients are safe and cost-effective, and intake thereof in the form of food or drink products or nutritious supplements can be easy.

### [Brief Description of the Drawings]

Fig. 1 is a graph showing effects of lactic acid bacteria for improving blood hemoglobin level.
Fig. 2 is a graph showing effects of lactic acid bacteria for improving renal functions. a) to c) show the changes in sodium ion, chloride ion and potassium ion, respectively.
Fig. 3 is a graph showing effects of lactic acid bacteria for ameliorating anemia. a) to c) show the changes in erythrocytes, hemoglobin and hematocrit, respectively.

### [Embodiments for Carrying out the Invention]

The present invention is based on finding that administration of lactic acid bacteria according to the invention can ameliorate renal anemia. Specifically, the present invention is based on the finding that administration of lactic acid bacteria according to the invention (*Lactobacillus gasseri* strain CP2305, Lactobacillus gasseri strain CP2305s or *Lactobacillus amylovorus* strain CP1563) to the subjects of middle-aged to older people (people in their 40s to 90s) would exert effects of improving hemoglobin level and others, compared with placebo samples, and the lactic acid bacteria would exert effects of ameliorating renal anemia.

Accordingly, described herein is a composition for use in ameliorating renal anemia comprising, as an active ingredient, lactic acid bacteria and/or a treated product thereof according to the invention.

The lactic acid bacteria used in the present invention are capable of producing lactic acid from sugars by fermentation. Bacterial cells of lactic acid bacteria that are not part of the claimed invention are also described herein and are known in the art and which may exert the function of ameliorating renal anemia specifically described below. In addition, the bacterial strains according to the invention have been confirmed to be safe for animals in terms of administration to or intake by animals.

Specific examples of lactic acid bacteria which are not part of the claimed invention, but which are described herein, include bacteria such as *Lactobacillus casei, Lactobacillus paracasei, Lactobacillus zeae, Lactobacillus rhamnosus, Lactobacillus reuteri, Lactobacillus acidophilus, Lactobacillus crispatus, Lactobacillus gallinarum, Lactobacillus brevis, Lactobacillus fermentum, Lactobacillus plantarum, Lactobacillus delbrueckii subsp. bulgaricus,* and *Lactobacillus johnsonii.*

Regarding the present invention, the function of ameliorating renal anemia is a function of ameliorating renal anemia in a subject. Renal anemia is anemia that occurs when erythropoietin, a hematopoietic hormone contributing to the production of erythrocytes, is not sufficiently produced due to decreased function of the kidneys, resulting in insufficient production of erythrocytes. For example, if the hemoglobin level is less than 12.7 g/dL for adult males and less than 11.0 g/dL for adult females and there is no anemic causative disease other than a decrease in erythropoietin production, it is diagnosed as renal anemia. Symptoms of renal anemia include palpitation, shortness of breath, dizziness, malaise and the like. Renal anemia is different from iron deficiency anemia caused by insufficient production of hemoglobin due to iron deficiency, but a subject may also suffer from both renal anemia and iron deficiency anemia. According to the present invention, the term "amelioration (ameliorating)" means ameliorating at least one symptom of renal anemia, increasing hemoglobin level and/or erythropoietin, approximating hemoglobin level and/or erythropoietin to normal values (regarding hemoglobin level, 12.7 to 16.4 g/dL for adult males, 11.0 to 14.8 g/dL for adult females; regarding erythropoietin, greater than 50 mIU/mL), as well as preventing the decrease of erythrocytes and/or erythropoietins.

Whether or not a bacterial cell or a treated product of lactic acid bacteria described herein, but which are not part of the claimed invention, exerts the function of ameliorating renal anemia can be evaluated by administering the bacterial cell or treated product of lactic acid bacteria belonging to the genus *Lactobacillus* to an experimental animal or a test volunteer, and confirming the amelioration of renal anemia (for example, measuring hemoglobin level, erythrocyte number, or hematocrit value, or determining the symptoms of renal anemia) before and after the administration.

Lactic acid bacteria according to the invention having the function according to the invention are *Lactobacillus gasseri* strain CP2305, *Lactobacillus gasseri* strain CP2305s, and *Lactobacillus amylovorus* strain CP1563. *Lactobacillus gasseri* strain CP2305 is deposited internationally by the applicant under Accession Number FERM BP-11331 as of September 11, 2007 with the International Patent Organism Depositary, the National Institute of Advanced Industrial Science and Technology (AIST) (Tsukuba Central 6, 1-1-1 Higashi, Tsukuba, Ibaraki, Japan), which is the international depositary authority under the terms of the Budapest Treaty for deposition of patent microorganisms. At present, this strain is transferred to and stored at the Patent Microorganisms Depositary, the National Institute of Technology and Evaluation (120, 2-5-8, Kazusakamatari, Kisarazu-shi, Chiba, Japan), which is the international depositary authority. Also, *Lactobacillus gasseri* strain CP2305s is deposited internationally by the applicant under Accession Number NITE BP-1405 as of August 14, 2012 with the Patent Microorganisms Depositary, the National Institute of Technology and Evaluation (122, 2-5-8, Kazusakamatari, Kisarazu-shi, Chiba, Japan), which is the international depositary authority under the terms of the Budapest Treaty for deposition of patent microorganisms. Further, *Lactobacillus amylovorus* strain CP1563 is deposited internationally by the applicant under Accession Number FERM BP-11255 as of May 25, 2010 with the International Patent Organism Depositary, the National Institute of Advanced Industrial Science and Technology (AIST), which is the international depositary authority under the terms of the Budapest Treaty for deposition of patent microorganisms. At present, this strain is transferred to and stored at the Patent Microorganisms Depositary, the National Institute of Technology and Evaluation (120, 2-5-8, Kazusakamatari, Kisarazu-shi, Chiba, Japan), which is the international depositary authority.

Described herein, but not part of the claimed invention, are variants of the specific strains mentioned above having the function of ameliorating renal anemia. *Lactobacillus gasseri* strain CP2305s according to the invention is a variant obtained from *Lactobacillus gasseri* strain CP2305 according to the invention. Variants of the strain CP2305 and the strain CP2305s as well as *Lactobacillus amylovorus* strain CP1563 are highly likely to have the function of ameliorating renal anemia.

The term "variant" used herein refers to any strain obtained from a parent strain. Specifically, this term refers to a strain obtained by a method of artificially increasing the frequency of mutation from a parent strain by means of spontaneous mutation or mutagenesis with chemical or physical mutagens or a strain obtained by a specific mutagenesis technique (e.g., gene recombination). Microbial individuals resulting from such techniques may be repeatedly subjected to selection and separation, and variants with the function of ameliorating renal anemia can be obtained by breeding of useful microbial individuals.

For example, variants originating from *Lactobacillus gasseri* strain CP2305 or CP2305s or *Lactobacillus amylovorus* strain CP1563 can be easily distinguished from other lactic acid bacterial strains based on the molecular weight distribution of amplified genomic DNA fragments of lactic acid bacteria determined by the polymerase chain reaction (PCR). In short, DNA samples of lactic acid bacteria of interest are prepared, gene amplification is carried out by PCR using primers having unique sequence (e.g., 16S rDNA-derived nucleotide sequence), and electrophoresis patterns of the obtained fragments are analyzed. Thus, whether or not the lactic acid bacterial strain of interest is a variant originating from *Lactobacillus gasseri* strain CP2305 or CP2305s or *Lactobacillus amylovorus* strain CP1563 can be determined. It should be noted that the technique of confirming whether or not the bacterial strain of interest is a variant is not limited to the above, and whether or not the bacterial strain of interest is a variant can be confirmed by a technique known in the art based on, for example, mycological properties.

Lactic acid bacteria can be prepared via culture under adequate conditions using any of media conventionally used for culture of the lactic acid bacteria. In this case, a natural or synthetic medium can be used, as long as it contains a carbon source, a nitrogen source, mineral salts, and the like, and lactic acid bacteria can be cultured efficiently therein. A person skilled in the art can adequately select a known medium suitable for a bacterial strain to be used. Examples of the carbon source that can be used include lactose, glucose, sucrose, fructose, galactose, and blackstrap molasses. Examples of the nitrogen source that can be used include organic nitrogen-containing substances such as casein hydrolysate, whey protein hydrolysate, and soy protein hydrolysate. In addition, examples of the mineral salts that can be used include phosphate, sodium, potassium, and magnesium. Examples of media suitable for culture of lactic acid bacteria include MRS liquid medium, GAM medium, BL medium, Briggs Liver Broth, animal milk, skim milk, and milk-derived whey. Preferably, sterilized MRS medium can be used.

Culture of lactic acid bacteria can be performed at 20°C to 50°C, preferably at 25°C to 42°C, and more preferably at approximately 37°C under anaerobic conditions. Temperature conditions can be adjusted using a thermostatic bath, a mantle heater, a jacket, or the like. The term "anaerobic conditions" used herein refers to a low-oxygen environment in which lactic acid bacteria can proliferate. For example, anaerobic conditions can be provided by using an anaerobic chamber, anaerobic box, or airtight container or bag containing a deoxidizer, or by simply sealing a culture container. Examples of culture formats include static culture, shake culture, and tank culture. The period of culture can be 3 hours to 96 hours. It may be preferable to maintain the pH of a medium at 4.0 to 8.0 in the beginning of culture.

Specific examples of lactic acid bacteria preparation are briefly described below. When *Lactobacillus gasseri* strain CP2305 or CP2305s or *Lactobacillus amylovorus* strain CP1563 is used, for example, lactic acid bacteria are inoculated into a medium for lactic acid bacteria culture (e.g., an MRS liquid medium) and preferably a food-grade medium for lactic acid bacteria culture, and culture may be carried out overnight (for approximately 18 hours) at approximately 37°C.

After culture, the obtained culture product of lactic acid bacteria can be used in that state, or it may be further subjected to, for example, crude purification via centrifugation and/or solid-liquid separation via filtration and sterilization, according to need. In addition, lactic acid bacteria used in the present invention may be in the form of viable bacterial cells or dead bacterial cells and/or in the form of wet bacterial cells or dried bacterial cells. Use of dead lactic acid bacteria may be preferable.

In addition, a treated product of lactic acid bacteria according to the invention obtained by treating bacterial cells of the lactic acid bacteria may be used, as long as it has the function of ameliorating renal anemia. Alternatively, the treated product of the lactic acid bacteria may be further subjected to treatment. Examples of such treatment are described below.

Bacterial cells and/or a treated product of lactic acid bacteria can be prepared in the form of a suspension or diluted solution by suspension or dilution in an adequate solvent. Examples of a solvent that can be used include water, physiological saline, and phosphate buffer saline (PBS).

A fermentation product can be prepared by fermenting raw milk, skim milk, or soymilk using bacterial cells and/or a treated product of lactic acid bacteria. For example, lactic acid bacteria or lactic acid bacteria subjected to optional treatment may be inoculated into raw milk, skim milk, or soymilk, followed by fermentation under conditions (substantially equivalent to the above conditions for culture of lactic acid bacteria) known in the art. The thus obtained fermentation product can be used in that state, or it may be subjected to optional treatment such as filtration, sterilization, dilution, or concentration.

A sterilized product of lactic acid bacteria can be prepared by sterilization treatment of bacterial cells and/or a treated product of lactic acid bacteria. In order to subject bacterial cells and/or a treated product of lactic acid bacteria to sterilization treatment, for example, a known technique of sterilization treatment such as filtration sterilization, radiation sterilization, heat sterilization, or high pressure sterilization can be used.

In addition, bacterial cells and/or a treated product of lactic acid bacteria can be subjected to heat treatment so as to prepare a heated product of lactic acid bacteria. In order to prepare such heated product, bacterial cells and/or a treated product of lactic acid bacteria are(is) subjected to high-temperature treatment (for example, at 80°C to 150°C) for a certain period of time, such as approximately 10 minutes to 1 hour (e.g., approximately 10 to 20 minutes).

Bacterial cells and/or a treated product of lactic acid bacteria can be subjected to disruption, fracturing, or grinding to prepare a disrupted product or a cell-free extract. As used herein, the term "disrupted cells" includes bacterial cells treated by means of disruption, grinding or milling, enzyme treatment, chemical treatment, or lysis. The "disrupted cells" may also include water-soluble fractions, organic solvent-soluble fractions, fractions hardly soluble in organic solvent or in water, or fractions insoluble in organic solvent or in water, which are obtained after the cells were disrupted. The disruption of bacterial cells can be performed by physical disruption, enzymatic lysis treatment, chemical treatment, or autolysis treatment using a method and an instrument known in the art.

Physical disruption may be carried out by using either a wet system (which is a treatment conducted in the state of a cell suspension) or a dry system (which is a treatment carried out in the state of a cell powder). The disruption of bacterial cells may be carried out by agitation using, for example, homogenizer, ball mill, bead mill, Dyne-Mill, or satellite mill, or by pressure application using, for example, jet mill, French press, or cell disruptor, or by filtration using a filter.

Enzymatic lysis treatment can be performed to break the cell structure of lactic acid bacteria using an enzyme, such as lysozyme.

Chemical treatment can be performed to break the cell structure of lactic acid bacteria using a surfactant, such as glycerin fatty acid ester or soybean phospholipid.

Autolysis treatment can be performed through lysis of cells with part of enzymes possessed by some lactic acid bacteria.

In the present invention, physical disruption may be preferable because the addition of other reagents or components is not needed. When physical disruption is carried out by agitation, for example, a cell suspension or cell powder may be agitated at 50 to 10,000 rpm, and preferably 100 to 1,000 rpm.

As a specific method for preparing disrupted cells, the bacterial cells are disrupted by, for example, treating a suspension of a lactic acid bacterium in a known Dyno-Mill cell disruptor (e.g., Dyne-Mill disruptor) using glass beads at a peripheral speed of 10.0 to 20.0 m/s (e.g., about 14.0 m/s) and a treating flow rate of 0.1 to 10 L/10 min (e.g., about 1 L/10 min) at a disruption tank temperature of 10°C to 30°C (e.g., about 15°C) 1 to 7 times (e.g., 3 to 5 times). Alternatively, a suspension of a lactic acid bacterium may be treated using a known wet-type jet-mill cell disruptor (e.g., JN20 Nano Jet Pul) at a discharge pressure of 50 to 1000 Mpa (e.g., 270 Mpa) at a treating flow rate of 50 to 1000 ml/min (e.g., 300 ml/min) 1 to 30 times (e.g., 10 times) to disrupt the bacterial cells. Further, lactic acid bacterial cell powder may be treated using a known dry-type satellite mill cell disruptor (e.g., GOT5 Galaxy 5) in the presence of any of different balls (e.g., 10-mm zirconia balls, 5-mm zirconia balls, or 1-mm alumina balls) at 50 to 10,000 rpm (e.g., 240 rpm or 190 rpm) for 30 minutes to 20 hours (e.g., 5 hours), thereby being able to disrupt the bacterial cells. Lactic acid bacterial cell powder may also be treated using a known dry-type jet-mill cell disruptor (e.g., Jet-O-Mizer) at a feeding speed of 0.01 to 10000 g/min (e.g., 0.5 g/min) and a discharge pressure of 1 to 1,000 kg/cm² (e.g., 6 kg/cm²) 1 to 10 times (e.g., once), thereby disrupting the bacterial cells.

According to the present invention, although the disrupted cells of lactic acid bacteria according to the invention exert the effect even when the cells are merely perforated, it is preferable to prepare the disrupted cells so that the average long diameter of the cells of lactic acid bacteria is 90% or less of that of before disruption treatment. When cells are disrupted via lysis treatment, for example, the average long diameter of the cells may occasionally be 0%. As such, the lactic acid bacteria may be disrupted so that the average long diameter of disrupted cell products is 0% to 90%, preferably 0% to 80%, 0% to 70%, 0% to 50%, and more preferably 0% to 20% of that of before disruption. The average long diameter of the disrupted cells of lactic acid bacteria varies depending on the type of the lactic acid bacteria to be used. For example, it may be 0 to 2.5 µm, preferably 0 to 2 µm, more preferably 0 to 1.5 µm, 0 to 1 µm, and further preferably 0 to 0.5 µm. The average long diameter can be calculated by, for example, measuring long diameter of 100 or more cells (including debris form) and obtaining average as the average long diameter.

Bacterial cells and/or a treated product of lactic acid bacteria can be subjected to extraction with the use of an adequate aqueous or organic solvent to obtain an extract. A method of extraction is not particularly limited, as long as such method involves the use of an aqueous or organic solvent as an extraction solvent. For example, a known method, such as a method comprising immersing the lactic acid bacteria or lactic acid bacteria subjected to optional treatment in an aqueous or organic solvent (e.g., water, methanol, or ethanol), or agitating or refluxing the lactic acid bacteria or lactic acid bacteria subjected to optional treatment in the solvent can be adopted.

Also, bacterial cells and/or a treated product of lactic acid bacteria can be subjected to drying to process into the form of a powdery product (powder) or granular product. Specific examples of drying methods include, but are not particularly limited to, spray drying, drum drying, vacuum drying, and freeze-drying, which can be used alone or in combination. Upon drying, a conventional excipient may be added, according to need.

In addition, bacterial cells and/or a treated product of lactic acid bacteria can be subjected to known separation/purification techniques to purify an ingredient or fraction having the function of ameliorating renal anemia. Examples of such separation/purification techniques include: a method involving salt precipitation or organic solvent precipitation in accordance with degrees of solubility; a method involving dialysis, ultrafiltration, or gel filtration in accordance with molecular weight differences; a method involving ion-exchange chromatography in accordance with charge differences; a method involving affinity chromatography in accordance with degrees of specific binding; and a method involving hydrophobic chromatography or reversed-phase chromatography in accordance with degrees of hydrophobicity, which can be used alone or in combinations of two or more thereof.

The treatments described above may be carried out alone or in combinations of two or more thereof. Such treated products according to the invention can be used in the composition of the present disclosure.

Through continuous intake of the bacterial cells and/or a treated product of lactic acid bacteria according to the invention obtained above alone or in combination with other ingredients in the form of the composition of the present disclosure, food or drink products, feeds, nutritious supplements, or pharmaceutical compositions, effects of ameliorating renal anemia can be contemplated.

The composition according to the invention comprises, as an active ingredient, the bacterial cells and/or a treated product of lactic acid bacteria according to the invention. Such composition may comprise a single type of bacterial cells and/or a treated product of lactic acid bacteria according to the invention, a plurality of different types of bacterial cells and/or a treated product of lactic acid bacteria according to the invention, or treated products of a plurality of lactic acid bacteria according to the invention subjected to different treatments in combination.

In addition to the bacterial cells and/or a treated product of lactic acid bacteria according to the invention as active ingredients, the composition of the present disclosure may be supplemented with the additives described below, other known drugs, or nutritious supplements alone or in combinations of two or more, as long as the function of ameliorating renal anemia is not inhibited.

While the dosage form of the composition of the present disclosure is not particularly limited, examples of dosage forms include: oral formulations such as tablets, capsules, granules, powders, dust formulations, syrups, dry syrups, solutions, suspensions, and inhalers; enteral formulations such as suppositories; infusions; and parenteral injections. Among them, an oral formulation may be preferable. In addition, a liquid formulation, such as a solution or suspension, may be dissolved or suspended in water or a different adequate medium immediately before use. In the case of tablets or granules, surfaces thereof may be coated by a well-known method. Further, the composition of the present disclosure may be prepared in the form of a controlled-release formulation, such as a sustained-release formulation, a delayed-release formulation, or an immediate-release formulation, by a technique known in the art.

The composition in the dosage form described above can be prepared in accordance with a conventional method by formulating conventional additives, such as excipients, disintegrants, binders, wetting agents, stabilizers, buffering agents, lubricants, preservatives, surfactants, sweeteners, flavoring agents, aromatics, acidulants, and coloring agents, into the ingredients described above in accordance with the dosage form. When the composition of the present disclosure is prepared in the form of a pharmaceutical composition, for example, a pharmaceutically acceptable carrier or additive can be incorporated into the composition. Examples of such pharmaceutically acceptable carriers and additives include water, pharmaceutically acceptable organic solvents, collagen, polyvinyl alcohol, polyvinyl pyrrolidone, carboxyvinyl polymers, sodium alginate, water-soluble dextran, water-soluble dextrin, sodium carboxymethyl starch, pectin, xanthan gum, gum Arabic, casein, gelatin, agar, glycerin, propylene glycol, polyethylene glycol, vaseline, paraffin, stearyl alcohol, stearic acid, human serum albumin, mannitol, sorbitol, lactose, surfactants acceptable as pharmaceutical additives, and artificial cell constructs such as liposomes.

When the composition of the present disclosure comprises the additives described above or other drugs, the content of the bacterial cells and/or a treated product of lactic acid bacteria according to the invention as active ingredients varies depending on the dosage form. The content of the lactic acid bacteria may be generally 0.0001% to 99% by mass, preferably 0.001% to 80% by mass, and more preferably 0.001% to 75% by mass. It may be preferable that the composition be prepared into a dosage form that allows management of the daily dose, so as to achieve intake of the active ingredients in preferable amounts. In addition, the number of the lactic acid bacteria or a treated product thereof to be incorporated into the composition of the present disclosure may be approximately 10⁷ cells/g to approximately 10¹² cells/g (when counted as the number of bacterial cells of lactic acid bacteria before treatment).

Examples of other drugs that can be added to or incorporated into the composition of the present disclosure include, but are not limited to, erythropoiesis stimulating agent (ESA).

The composition of the present disclosure may further contain a variety of additives used for production of medicaments, food or drink products, and feeds and other various substances. Examples of such substances and additives include a variety of fats and oils (e.g., plant oils, such as soybean oil, corn oil, safflower oil, and olive oil, and animal fats and oils, such as beef fat and sardine oil), crude drugs (e.g., royal jelly and ginseng), amino acids (e.g., glutamine, cysteine, leucine, and arginine), polyalcohols (e.g., ethylene glycol, polyethylene glycol, propylene glycol, glycerin, and sugar alcohols such as sorbitol, erythritol, xylitol, maltitol, and mannitol), natural polymers (e.g., gum Arabic, agar, water-soluble corn fibers, gelatin, xanthan gum, casein, gluten or gluten hydrolysate, lecithin, starch, and dextrin), vitamins (e.g., vitamin C and B-complex vitamins), minerals (e.g., calcium, magnesium, zinc, and iron), dietary fibers (e.g., mannan, pectin, and hemicellulose), surfactants (e.g., glycerin fatty acid esters and sorbitan fatty acid esters), purified water, excipients (e.g., glucose, cornstarch, lactose, and dextrin), stabilizers, pH-adjusting agents, antioxidants, sweeteners, taste components, acidulants, coloring agents, and flavors.

The amount of such additive can be adequately determined depending on the type of additive and the desirable amount to be intaken. The content of bacterial cells and/or a treated product of lactic acid bacteria according to the invention as active ingredients may vary depending on the dosage form. The content may be generally 0.0001% to 99% by mass, preferably 0.001% to 80% by mass, and more preferably 0.001% to 75% by mass, as the amount of the lactic acid bacteria before treatment.

Subjects of administration or intake of the composition of the present disclosure may be vertebrate animals. Specific examples thereof include mammals such as humans, primates (e.g., monkeys and chimpanzees), livestock animals (e.g., cattle, horses, pigs, sheep, and chickens), pet animals (e.g., dogs and cats), and experimental animals (e.g., mice and rats). Further, subjects can be reptiles and birds (e.g., chickens). Particularly preferable subjects may be those for whom renal anemia is expected to be ameliorated, such as humans having decreased hematopoietic function of the kidney, and humans having hemoglobin level of 13.5 g/dL or less for adult males and 11.5 g/dL or less for adult femals.

The dose of administration or intake of the composition of the present disclosure may vary depending on the age and the body weight of a subject, the administration/intake route, the administration/intake frequency, the purpose of administration, and other conditions. The dose of administration or intake can be changed extensively at the discretion of a person skilled in the art to achieve the effect of ameliorating renal anemia of interest. For oral administration or intake, for example, it may be preferable to administer bacterial cells and/or a treated product of lactic acid bacteria according to the invention contained in the composition in an amount of generally approximately 10⁶ cells to 10¹² cells, and preferably approximately 10⁷ cells to 10¹¹ cells per kg of body weight, as the amount of the bacterial cells of lactic acid bacteria. The content of bacterial cells and/or a treated product of lactic acid bacteria according to the invention is not particularly limited and can be adequately adjusted in accordance with the ease of production, the preferable daily dose, or other conditions. Since the composition of the present disclosure is highly safe, it is also possible to further increase the dose to be administered. A daily dose may be administered in a single instance or in several separate instances. In addition, the frequency of administration or intake is not particularly limited, and it can be adequately selected depending on various conditions, such as the route of administration or intake, age or body weight of a subject, and desired effects.

The administration or intake route of the composition of the present disclosure is not particularly limited, and it can be, for example, oral administration or intake or parenteral administration (e.g., intrarectal, subcutaneous, intramuscular, or intravenous administration). Particularly preferably, the composition of the present disclosure may be orally administered or ingested.

The composition of the present disclosure may probably improve the decreased renal function, and thereby effectively ameliorate renal anemia. Specifically, hemoglobin level, erythrocyte number or hematocrit level may be increased or the decrease thereof may be prevented in the subject, compared with a control to which the composition of the present disclosure (lactic acid bacteria and/or a treated product thereof) is not administered. Thus, the composition of the present disclosure has effects of ameliorating renal anemia in a subject, in particular, in a human subject having decreased hematopoietic function of the kidney.

The composition of the present disclosure may be used in combination with an other medicament or an other therapeutic or preventive method. The "other medicament" and the composition of the present disclosure may be formulated into a single formulation. Alternatively, they may be formulated into separate formulations to administer them simultaneously or at intervals.

As described above, the composition of the present disclosure can be used in the form of a pharmaceutical composition for ameliorating renal anemia, and other purposes.

In addition, the composition of the present disclosure is highly safe and thus is easily used for long-term continuous intake. Therefore, the composition of the present disclosure can also be added to food or drink products, nutritious supplements, or feeds. The composition of the present disclosure has the function described above, and it contains lactic acid bacteria according to the invention that have been used for meals. Thus, such composition is highly safe. Further, even when it is added to a variety of food or drink products, it does not inhibit the flavor of a food or drink product itself. Thus, it can be added to various types of food or drink products and continuously ingested. As a consequence, the function of ameliorating renal anemia of interest can be expected.

The food or drink product described herein contains the composition of the present disclosure as described above. The food or drink product described herein also includes beverages. Examples of the food or drink product containing the composition of the present disclosure include functional food or drink products aimed at better health by the amelioration of renal anemia (e.g., nutritious supplements, food for specified health use, foods with nutrient function claims, and foods with functional claims), and all food or drink products into which the composition of the present disclosure can be incorporated.

Functional food or drink products are particularly preferable as food or drink products containing the composition of the present disclosure. The "functional food or drink product" described herein means a food or drink product having a predetermined function for organisms and encompasses, for example, all of so-called health food or drink products such as food or drink products with health claims including foods for specified health use (FOSHU) and food or drink products with nutrient function claims, foods with function claims, foods for special dietary uses, nutritional supplements, health supplements, supplements (e.g., those having a variety of dosage forms such as tablets, coated tablets, sugar-coated tablets, capsules, and liquid agents), and beauty food or drink products (e.g., diet food or drink products). The functional food or drink products described herein also encompass health food or drink products to which health claim based on Codex (Joint FAO/WHO Food Standards Programme) food standards are applied.

Specific examples of food or drink products include: health food or drink products and nutritional supplements in preparation forms such as liquid diets (e.g., tube enteral nutritional supplements), tablet candies, tablets, chewable tablets, tablets, dust formulations, powders, capsules, granules, and tonic drinks; tea beverages such as green tea, oolong tea, and black tea; beverage products such as soft drinks, jelly beverages, sport beverages, milk beverages, carbonated beverages, vegetable beverages, juice beverages, fermented vegetable beverages, fermented fruit juice beverages, fermented milk beverages (e.g., yogurt), lactic acid bacteria beverages, milk beverages (e.g., coffee milk and fruit milk), beverages containing drink powders (e.g., milk formula), cocoa beverages, milk, and purified water; spreads such as butter, jam, dried seasoning products, and margarine; mayonnaise; shortening; custard; dressings; breads; boiled rice; noodles; pasta; Japanese *miso* soup; Japanese *tofu;* yogurt; baby foods; soups or sauces; and sweets (e.g., biscuits and cookies, chocolates, candies, cakes, ice creams, chewing gums, and tablets).

The food or drink product described herein can be produced by conventional methods by adding other food materials used for production of the above-mentioned food or drink products, various nutrients, various vitamins, minerals, dietary fibers, and various additives (e.g., taste components, sweeteners, acidulants such as organic acids, stabilizers, and flavors), in addition to the above-mentioned active ingredients.

For the food or drink product described herein, a person skilled in the art can adequately determine the amount of the lactic acid bacteria and/or a treated product thereof according to the invention as an active ingredient to be incorporated thereinto by taking the form of the food or drink product and the desirable taste or texture into consideration. In general, a person skilled in the art may appropriately determine the total amount of bacterial cells and/or a treated product of lactic acid bacteria to be added to the composition, so as to adjust the amount of lactic acid bacteria to 0.0001% to 99% by mass, preferably 0.001% to 80% by mass, and more preferably 0.001% to 75% by mass. The composition of the present disclosure is highly safe. As such, the amount of the composition incorporated into a food or drink product can be further increased. In order to achieve consumption of the desirable amount of the composition, it may be desirable to prepare the composition in a dosage form that allows management of the daily dose. As described above, the food or drink product described herein can be consumed in a manner that allows management of a desirable amount of the composition of the present disclosure. Thus, ameliorating renal anemia using such food or drink products is described.

The composition of the present disclosure may be incorporated into a food or drink product by any appropriate method available to a person skilled in the art. For example, the composition of the present disclosure can be prepared in the liquid, gel, solid, powdery, or granular form and the resultant is then incorporated into the food or drink product. Alternatively, the composition of the present disclosure may be mixed or dissolved directly into raw materials of the food or drink product. The composition of the present disclosure may be applied to, coated onto, infiltrated into, or sprayed onto the food or drink product. The composition of the present disclosure may be dispersed uniformly or distributed unevenly in the food or drink product. A capsule or the like containing the composition of the present disclosure may be prepared. The composition of the present disclosure may be wrapped with an edible film or a food coating agent. Alternatively, an adequate excipient or the like may be incorporated into the composition of the present disclosure, and the resultant may be prepared in the form of, for example, a tablet. The food or drink product containing the composition of the present disclosure may further be processed. Such a processed product also falls within the scope of the present invention.

The food or drink product described herein may be prepared with the use of various types of additives that are commonly used for food or drink products.

As described above, the food or drink product described herein has useful effects, it is highly safe, and thus there is no concern about side effects. Further, the composition of the present disclosure has a favorable flavor. Even when it is added to a variety of food or drink products, it does not inhibit the flavor of the food or drink product. Accordingly, the resulting food or drink product can be easily subjected to long-term continuous ingestion, and useful effects thereof can be expected for a long period of time.

Further, the composition of the present disclosure can be formulated not only into food or drink products for humans but also into feeds for animals such as livestock (e.g., cattle, pigs, and chickens), racehorses, and pets (e.g., dogs and cats). Feeds are substantially equivalent to food or drink products except that they are given to non-human subjects. Therefore, the descriptions concerning food or drink products above can also be applied to feeds.

Hereafter, the present invention is described in greater detail with reference to the examples, although the present invention is not limited to these examples.

### [Test Example]

### (1) Preparation of lactic acid bacteria

As lactic acid bacteria, *Lactobacillus gasseri* strain CP2305 (Accession Number: FERM BP-11331) and *Lactobacillus gasseri* strain CP2305s (Accession Number: NITE BP-1405) were prepared.

### (2) Measurement of hemoglobin level

Blood was collected from subjects, and hemoglobin level was measured by Sodium lauryl sulfate-Hemoglobin (SLS-Hb) method.

### (3) Renal function (Na⁺Cl⁻ excretion)

Using the blood serum from the subjects, renal function was measured conventionally by electrode method.

### (4) Measurement of erythrocytes, hemoglobin level, and hematocrit

Blood was collected from the subjects, and the number of erythrocytes and the hematocrit level were measured by electric resistance detection method, and hemoglobin level was measured by Sodium lauryl sulfate-Hemoglobin (SLS-Hb) method.

### [Example 1]

### <Preparation of test beverage>

With the use of *Lactobacillus gasseri* strain CP2305 and *Lactobacillus helveticus* strain, skimmed milk powder and an yeast extract were fermented at 32°C to 37°C for 12 to 22 hours, the resulting fermented milk was supplemented with liquid sugar, a sweetener, an acidulant, a stabilizer, and a flavoring agent, and the resultant was then sterilized.

A placebo beverage was prepared and sterilized in the same manner as described above with the use of milk fermented with *Lactobacillus helveticus* strain under the same conditions while refraining from the use of the strain CP2305.

A paper bottle was filled with 200 ml of the sterilized milk, and the test beverage and the placebo beverage were prepared.

### [Example 2]

### <Test of beverage for human use>

Subjects (34 subjects, middle-aged to older people in their 40s to 90s) were divided into two groups, a bottle of the test beverage containing *Lactobacillus gasseri* strain CP2305 (the amount of bacterial cells: 10¹⁰ cells) or the placebo beverage that does not contain the strain CP2305 was administered every day over the period of 12 weeks.

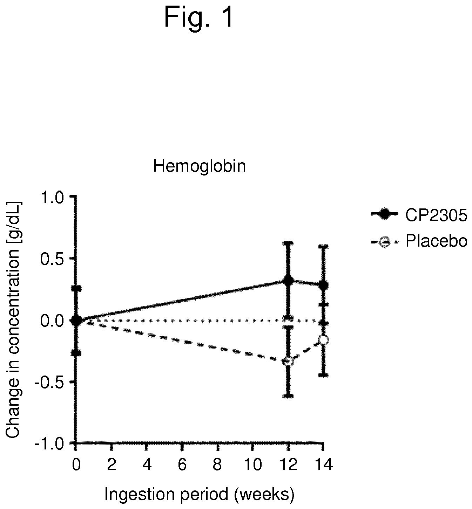

As a result, the increasing tendency in hemoglobin level, a blood indicator of anemia, was observed in the group to which the strain CP2305 had been administered (Fig. 1). Fig. 1 shows the changes in the hemoglobin level relative to a period of drinking (weeks).

### [Example 3]

### <Preparation of test beverage>

*Lactobacillus amylovorus* strain CP1563 was cultured in a culture medium for food additives using glucose, peptone, yeast extract, sodium acetate, dipotassium hydrogenphosphate, magnesium sulfate, glycerin fatty acid ester at 37°C for 24 hours. Then, the strain was collected, washed, sterilized, lyophilized, and subjected to a treatment using FS-4 Jet Mill (SEISHIN ENTERPRISE Co., Ltd.) under the pressure of 0.64 MPa at 0.5 kg/h to obtain disrupted cells. Next, the obtained CP1563 disrupted cells were mixed with skimmed milk powder, an acidulant, a sweetener, a stabilizer, an emulsifying agent, and a flavoring agent, and the resultant was then sterilized. The resultant was filled in to prepare a test beverage.

### [Example 4]

### <Test of beverage for human use>

A placebo-controlled double-blind comparative study was conducted for subjects (200 subjects, BMI: 25 to 30 kg/m²). The subjects ingested 500 ml of the beverage containing disrupted cells of *Lactobacillus amylovorus* strain CP1563 (200 mg) or the placebo beverage that does not contain the strain CP1563 every day over the period of 12 weeks. The measurements were conducted at 0, 4, 8 and 12 weeks.

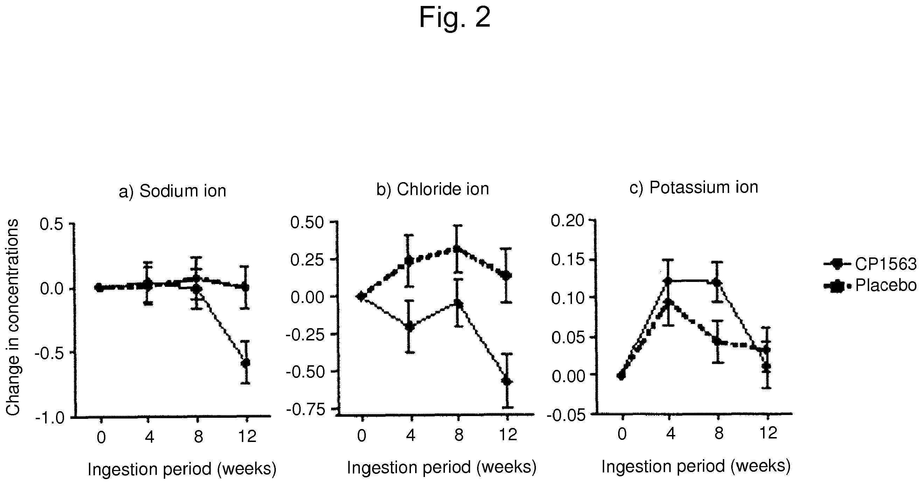

Fig. 2 shows the changes in the concentrations of sodium ion, chloride ion and potassium ion, respectively, relative to a period of drinking (weeks). The organ responsible for the regulation of these electrolytes is the kidney, and it seems that lactic acid bacteria (the strain CP1563) controls the secretion of hormones which control the excretion and reabsorption of monovalent ions in the kidney.

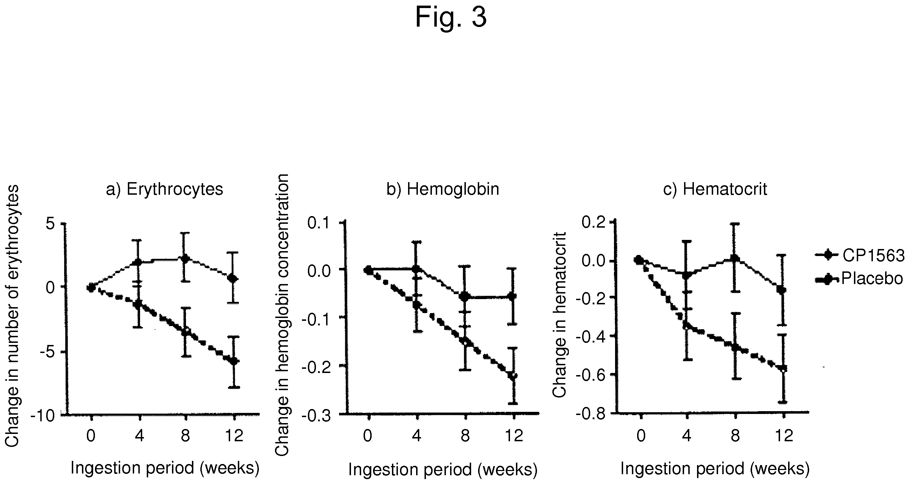

Fig. 3 shows the changes in the number of erythrocytes, hemoglobin level and hematocrit level, respectively, relative to a period of drinking (weeks). These indicators relating to the erythrocyte status are maintained by the ingestion of lactic acid bacteria (the strain CP1563), suggesting that lactic acid bacteria (especially, the strain CP1563) are involved in the control of hematopoietic hormones produced in the kidney.

### [Accession Numbers]

Accession Number: FERM BP-11331 (*Lactobacillus gasseri* strain CP2305; date of deposition: September 11, 2007)
Accession Number: NITE BP-1405 (*Lactobacillus gasseri* strain CP2305s; date of deposition: August 14, 2012)
Accession Number: FERM BP-11255 (*Lactobacillus amylovorus* strain CP1563; date of deposition: May 25, 2010)

## Claims

1. A composition for use in ameliorating renal anemia in a subject having decreased hematopoietic function of the kidney or a subject having decreased hemoglobin comprising, as an active ingredient, lactic acid bacteria, wherein the lactic acid bacteria are *Lactobacillus gasseri* strain CP2305 (Accession Number: FERM BP-11331), *Lactobacillus gasseri* strain CP2305s (Accession Number: NITE BP-1405), or *Lactobacillus amylovorus* strain CP1563 (Accession Number: FERM BP-11255), and/or a treated product thereof; wherein the treated product thereof comprises disrupted cells or cell-free extract of the lactic acid bacteria.

2. The composition for use according to claim 1, wherein the lactic acid bacteria are dead cells.

3. The composition for use according to claim 1 or claim 2, wherein the treated product of lactic acid bacteria is disrupted cells.

4. The composition for use according to any one of claims 1 to 3, which is selected from the group consisting of food or drink products, nutritious supplements, and medicaments.

5. The composition for use according to claim 4, wherein the food or drink products comprise fermented milk beverages, yogurt, powdered milk, baby foods, *miso* soup, retort foods, and tablets.

6. The composition for use according to claims 1 to 5, wherein the subject has decreased hematopoietic function of the kidney.

## Patentansprüche

1. Zusammensetzung zur Verwendung bei der Linderung von renaler Anämie in einem Subjekt mit verminderter hämatopoetischer Funktion der Niere oder einem Subjekt mit vermindertem Hämoglobin, umfassend, als Wirkstoff, Milchsäurebakterien, wobei es sich bei den Milchsäurebakterien um *Lactobacillus* gasseri-Stamm CP2305 (Zugangsnummer: FERM BP-11331), *Lactobacillus* gasseri-Stamm CP2305s (Zugangsnummer: NITE BP-1405) oder *Lactobacillus amylovorus-Stamm* CP1563 (Zugangsnummer: FERM BP-11255) und/oder ein behandeltes Produkt davon handelt, wobei das behandelte Produkt davon aufgebrochene Zellen oder einen zellfreien Extrakt der Milchsäurebakterien umfasst.

2. Zusammensetzung zur Verwendung nach Anspruch 1, wobei es sich bei den Milchsäurebakterien um tote Zellen handelt.

3. Zusammensetzung zur Verwendung nach Anspruch 1 oder Anspruch 2, wobei es sich bei dem behandelten Produkt der Milchsäurebakterien um aufgebrochene Zellen handelt.

4. Zusammensetzung zur Verwendung nach einem der Ansprüche 1 bis 3, ausgewählt aus der aus Nahrungsmittel- und Getränkeprodukten, Nahrungsergänzungsmittel und Medikamenten bestehenden Gruppe.

5. Zusammensetzung zur Verwendung nach Anspruch 4, wobei die Nahrungsmittel- und Getränkeprodukte Getränke aus fermentierter Milch, Jogurt, Milchpulver, Babynahrung, Misosuppe, Retortennahrungsmittel und Tabletten umfassen.

6. Zusammensetzung zur Verwendung nach einem der Ansprüche 1 bis 5, wobei das Subjekt an einer verminderten hämatopoetischen Funktion der Niere leidet.

## Revendications

1. Composition pour une utilisation dans l'amélioration de l'anémie rénale chez un sujet ayant une fonction hématopoïétique du rein diminuée ou un sujet ayant une hémoglobine diminuée comprenant, en tant qu'ingrédient actif, des bactéries d'acide lactique, les bactéries d'acide lactique étant la souche de *Lactobacillus gasseri* CP2305 (Numéro d'accès : FERM BP-11331), la souche de *Lactobacillus gasseri* CP2305s (Numéro d'accès : NITE BP-1405), ou la souche de *Lactobacillus amylovorus* CP1563 (Numéro d'accès : FERM BP-11255), et/ou un produit traité correspondant ; le produit traité correspondant comprenant des cellules dissociées ou un extrait exempt de cellules des bactéries d'acide lactique.

2. Composition pour une utilisation selon la revendication 1, les bactéries d'acide lactique étant des cellules mortes.

3. Composition pour une utilisation selon la revendication 1 ou la revendication 2, le produit traité de bactéries d'acide lactique étant des cellules dissociées.

4. Composition pour une utilisation selon l'une quelconque des revendications 1 à 3, qui est choisie dans le groupe constitué par des produits alimentaires ou à boire, des suppléments nutritionnels, et des médicaments.

5. Composition pour une utilisation selon la revendication 4, les produits alimentaires ou à boire comprenant des boissons au lait fermenté, un yaourt, le lait en poudre, des produits alimentaires pour bébés, une soupe *miso,* des produits alimentaires stérilisés, et des comprimés.

6. Composition pour une utilisation selon les revendications 1 à 5, le sujet ayant une fonction hématopoïétique du rein diminuée.
